# FASCICULE DE BREVET EUROPEEN

(11) **EP 3 080 510 B1**
(45) Date de publication et mention de la délivrance du brevet: **03.01.2018**
(21) Numéro de dépôt: 14821754.0
(22) Date de dépôt: 28.11.2014
(51) Int. Cl.: F17C 1/00, F17C 13/04

(54) **SYSTÈME DE STOCKAGE ET DE DISTRIBUTION D'UN MÉLANGE GAZEUX NO/AZOTE**
SYSTEM ZUR SPEICHERUNG UND VERTEILUNG EINES NO/STICKSTOFF-GASGEMISCHES
SYSTEM FOR STORING AND DISTRIBUTING AN NO/NITROGEN GASEOUS MIXTURE

(30) Priorité: 12.12.2013 FR 1362490
(43) Date de publication de la demande: 19.10.2016
(73) Titulaire: Air Liquide Santé (International), 75007 Paris (FR)
(72) Inventeur: DE VILLEMEUR, Pierre, F-78430 Louveciennes (FR); LECOURT, Laurent, F-94230 Cachan (FR); BESÈME, Catherine, F-75017 Paris (FR)
(74) Mandataire: Pittis, Olivier
(86) Numéro de dépôt international: PCT/FR2014/053084
(87) Numéro de publication internationale: WO 2015/086946

(56) Documents cités:
- EP-A1- 2 541 120
- EP-A2- 1 515 080
- WO-A1-99/49921
- WO-A2-01/41856
- DE-A1- 19 744 047

## Description

L'invention concerne un système de stockage et de distribution d'un mélange gazeux formé de NO/N₂ comprenant un robinet à détendeur intégré ou « RDI » en acier inoxydable monté sur un récipient de conditionnement de mélange gazeux de NO et d'azote à concentration d'au moins 200 ppmv (ppm en volume) et pouvant aller jusqu'à 3500 ppmv, et à une pression de 100 à 500 bar.

Le NO gazeux est classiquement utilisé à différentes concentrations allant de 200 à 1000 ppm en volume (ci-après « ppmv »), le reste du mélange gazeux étant de l'azote, pour traiter les vasoconstrictions pulmonaires, notamment l'hypertension pulmonaire chez des patients subissant une opération de chirurgie cardiaque ou chez des nouveau-nés hypoxiques. A ce titre, on peut citer les documents EP-A-786264 et EP-1516639.

Le mélange gazeux NO/azote est généralement conditionné à une pression de l'ordre de 100 à 200 bar absolus dans un récipient, telle une bouteille de gaz, équipé d'un bloc robinet permettant de contrôler la sortie du gaz dudit récipient.

Compte tenu de sa pression très élevée, le gaz doit subir une détente avant son administration au patient de manière à réduire sa pression et la rendre compatible avec une administration par inhalation.

Pour ce faire, on utilise un détendeur de gaz qui est soit fixé en sortie du robinet, soit intégré à un dispositif d'administration et monitorage du NO, situé en aval du robinet en étant relié à celui-ci par un flexible type haute pression.

Le document WO-A-99/49921 enseigne une installation de ce type avec bouteille de NO équipée d'un robinet de distribution du gaz et d'un détendeur de gaz externe fixé en sortie de robinet.

Or, ces systèmes posent certains problèmes.

Ainsi, lorsque le détendeur est relié au robinet par un flexible, ce flexible engendre un risque de blessure pour les utilisateurs du fait de la haute pression qu'il véhicule puisqu'il peut être source de mauvaises manipulations.

De plus, un volume de gaz non négligeable peut être contenu dans un flexible renfermant du gaz à haute pression, ce qui conduit potentiellement à une formation de NO₂ et donc nécessite de réaliser une purge régulière du flexible.

Dans le cas où le détendeur est fixé sur le raccord de sortie du robinet, se pose le problème de l'encombrement car ce détendeur est un élément supplémentaire venant se connecter sur le robinet, qui peut par exemple gêner les utilisateurs.

De plus, se pose aussi un risque lié à la haute pression du gaz, lors de l'installation du détendeur sur la bouteille. L'utilisation du mélange gazeux à base de NO doit pouvoir s'effectuer très rapidement. De là, soit on laisse le détendeur à demeure sur la bouteille, ce qui engendre un risque de dommages en cas de chute de la bouteille, soit on l'installe après coup mais, dans ce cas, le temps d'installation retardera le démarrage du traitement.

On connait aussi de EP-A-2541120, un système de stockage et de distribution de NO/N₂ comprenant un récipient de conditionnement du mélange gazeux NO/azote, équipé d'un robinet à détendeur intégré (RDI) permettant de contrôler le débit et la pression du gaz en sortie.

Ce type de système permet de résoudre certains des problèmes susmentionnés mais ne règle pas celui de la compatibilité des matériaux constituant le RDI avec les mélanges stockés et, dans certains cas, on a pu constater en pratique à une dégradation du passage véhiculant le gaz et/ou des éléments de détente, en particulier du clapet et/ou du siège de clapet.

De plus, ce type de RDI ne permet pas d'ajuster le débit de sortie indépendamment de la pression, et inversement.

Des dispositifs similaires ou analogues sont enseignés par les documents WO-A-41856, EP-A-1515080 et DE-A-19744047.

Le problème à résoudre est alors de pouvoir réaliser une réduction de la pression du mélange gazeux à base de NO sans rencontrer tout ou partie des problèmes susmentionnés.

La solution est un système de stockage et de distribution d'un mélange gazeux formé de NO/N₂ comprenant :
- un récipient de conditionnement contenant un mélange gazeux formé de NO et d'azote, c'est-à-dire mélange gazeux NO/azote, typiquement le mélange gazeux NO/N₂ contient de 200 à 3500 ppm en volume de NO et de l'azote pour le reste, et
- un robinet à détendeur intégré (RDI) comprenant un corps de robinet en métal comprenant une entrée de gaz, au moins une sortie de gaz et au moins un passage interne gaz traversant ledit corps de robinet pour relier fluidiquement l'entrée de gaz à ladite au moins une sortie de gaz, ledit robinet à détendeur intégré étant fixé sur le récipient de conditionnement,
caractérisé en ce que :
- au moins la partie du corps de robinet traversée par ledit passage interne de gaz est en acier inoxydable, et
- le robinet à détendeur intégré comprend en outre :
   - des moyens de réglage de la détente du gaz coopérant avec un clapet de détente, ledit clapet coopérant avec un siège de clapet, le clapet de détente et le siège de clapet étant en acier inoxydable,
   - un premier raccord de sortie à basse pression qui délivre du gaz à une basse pression comprise entre 1 et 10 bars, et
   - un second raccord de sortie de débit associé à un dispositif débitlitre comprenant des orifices calibrés et un organe de sélection de débit pour permettre une délivrance du mélange gazeux NO/azote à plusieurs débits de gaz différents, c'est-à-dire que le gaz est délivré à un débit donné sélectionnable parmi plusieurs débits différents.

Grâce à la présente invention, les utilisateurs ne sont plus exposés à la haute pression gazeuse car le gaz est détendu directement dans le robinet à détente intégrée et sort donc à basse pression, c'est-à-dire typiquement entre 1 et 10 bar abs, par exemple entre 2 et 7 bar abs.

La basse pression peut être fixée à un niveau souhaité permettant ainsi un bon fonctionnement du système de délivrance de NO alimenté par le gaz détendu.

De plus, ceci évite aussi les risques de mauvaises manipulations existants avec les robinets antérieurs et la sortie basse pression du robinet-détendeur peut être connectée directement à un système d'administration et monitorage du NO.

De plus, la solution de l'invention permet également d'obtenir une réduction de l'encombrement global car aucun ajout d'un détendeur supplémentaire en sortie de robinet n'est plus nécessaire.

Enfin, la solution de l'invention permet aussi une délivrance de NO à différents débits de manière à pouvoir s'adapter à une situation particulière, comme une ventilation avec ventilateur d'administration de gaz ou, à l'inverse, sans ventilateur, par exemple en cas d'urgence.

Selon le cas, le système de l'invention peut comprendre l'une ou plusieurs des caractéristiques techniques suivantes :
- le corps est partiellement ou entièrement réalisé en acier inoxydable. Le fait d'utiliser de l'acier inoxydable en tant que matériau constitutif de tout ou partie des passages et des autres éléments en contact avec le gaz à base de NO, en particulier le clapet et le siège de clapet, est avantageux car cela permet d'éviter ou de minimiser la corrosion des éléments du robinet-détendeur du fait de la nature corrosive du NO/gaz.
- le récipient de conditionnement est de forme cylindrique.
- le récipient de conditionnement comprend un fond et un col avec un orifice de sortie au niveau duquel est fixé le robinet détendeur intégré.
- le robinet à détendeur intégré est protégé par un capotage de protection.
- un marquage ou une coloration donnée permet d'identifier la concentration de NO.
- un système de détrompage permet d'éviter les risques d'erreur de raccordement au robinet à détendeur intégré (RDI), c'est-à-dire au premier raccord de sortie à basse pression du RDI .
- le robinet à détendeur intégré comprend en outre des moyens de contrôle de la libération du gaz.
- les moyens de réglage de la détente du gaz et/ou les moyens de contrôle de la libération du gaz comprennent un ou des organes rotatifs actionnables manuellement par un utilisateur, tel qu'un (ou plusieurs) volant rotatif ou un levier pivotant.
- le récipient de conditionnement à un volume interne inférieur ou égal à 20 litres (équivalent en eau), de préférence un volume interne inférieur ou égal à 15 litres (équivalent en eau) et supérieur ou égale à 0,5 litres (équivalent en eau).
- il peut comporter un dispositif de suivi de l'autonomie de la bouteille en volume (litres) et/ou en temps (heures et/ou minutes).
- il comporte un dispositif de suivi de l'autonomie de la bouteille conçu pour communiquer des informations d'autonomie, notamment une ou des informations d'autonomie, notamment de temps et/ou de volume, à un ou d'autres systèmes, notamment un appareil de délivrance, un ordinateur ou un serveur distant. Les informations d'autonomie peuvent être transmises à un appareil de délivrance par exemple par voie infrarouge, filaire, Bluetooth, GSM, GPRS ou autre.
- le premier raccord de sortie de gaz à basse pression du RDI délivre un gaz à une pression entre 1 et 10 bar absolus, typiquement entre 2 et 7 bars, de préférence de 3 à 5 bar.
- le premier raccord de sortie de gaz à basse pression du RDI comprend des moyens de détrompage, par exemple un profil particulier ou analogue.
- la délivrance de NO gazeux à plusieurs débits de gaz différents se fait via le second raccord de sortie de débit.
- l'organe de sélection de débit est un bouton rotatif.
- le second raccord de sortie de débit coopère avec le dispositif débitlitre pour permettre une délivrance de NO gazeux.
- l'organe de sélection de débit coopère avec le dispositif débitlitre pour sélectionner l'un desdits orifices calibrés dont le diamètre de passage de gaz correspond au débit de gaz souhaité.
- le gaz traverse l'orifice calibré dont le diamètre de passage de gaz correspond au débit de gaz souhaité, est traversé par le flux gazeux et est délivré par le second raccord de sortie de débit.
- les orifices calibrés sélectionnables pour permettre une délivrance de NO gazeux à différents débits donnés sont agencés en amont du second raccord de sortie de manière à ce que le gaz traverse l'un de ces orifices calibrés avant d'atteindre le second raccord de sortie par lequel le gaz est distribué.
- les orifices calibrés ont des calibres différents les uns des autres, en particulier des diamètres croissants, correspondant aux différentes valeurs de débits désirés.
- les orifices calibrés ont des diamètres croissants correspondant à des valeurs de débits désirés compris entre 50 ml/min et 5 l/min, typiquement entre 75 ml/min et 2 l/min, en particulier entre 100 ml/min et 1,5 l/min.
- le système à orifices calibrés peut être conçu pour délivrer uniquement quelques débits différents, par exemple 2 à 6 débits différents, de sorte de permettre une utilisation en mode dégradé ou « secours », en cas de défaillance d'un appareil de délivrance de NO connecté a la sortie basse pression du RDI. Dans ce mode « secours », les différents débits peuvent être fixés par exemple entre 200 et 250 ml/min pour des patients adultes ou des nouveaux nés ventilés avec des ventilateurs en mode OHF (oscillations hautes fréquences) et entre 100 et 150 ml/min pour les enfants ou nouveaux nés ventilés en ventilation conventionnelle. En effet, de telles valeurs de débit correspondent à une concentration de 2 à 20 ppmv de NO pour des volumes-minutes adaptés au patient considéré, à savoir de 2 à 30 litres par exemple.
- le système à orifices calibrés peut aussi être conçu pour délivrer davantage de débits différents, par exemple plus de 10 valeurs de débit, de sorte de pouvoir opérer une délivrance plus précise dans le cadre d'une utilisation sans appareil de délivrance de NO sophistiqué, par exemple une utilisation en véhicule d'urgence (e.g., ambulance ou hélicoptère). Dans ce cas, la partie débitmétrie du RDI est plus complète et comporte des orifices calibrés correspondant à par exemple 10 à 15 débits présélectionnés, voire plus de 15 débits, par exemple des débits compris entre 10 ml/min et 1 l/min, qui correspondent à des valeurs de débit permettant une administration de NO chez des patients adultes, enfants et nouveaux nés ventilés de manière invasive ou non invasive.
- le robinet comprenant de 2 à 20 orifices calibrés ayant des diamètres croissants, typiquement de 2 à 15 orifices calibrés.
- les orifices calibrés sont aménagés sur un disque rotatif agencé sur le passage du gaz en amont du second raccord de sortie à débit donné.
- le robinet à détendeur intégré comporte en outre un disque de rupture et/ou fusible thermique assurant une sécurité accrue d'utilisation en laissant le gaz sous pression s'échapper à l'atmosphère dans le cas où une combustion accidentelle, tel un incendie, se produit dans le local de stockage de la bouteille de gaz équipée dudit RDI.

L'invention concerne aussi une installation de distribution d'un gaz contenant du NO à un patient comprenant :
- un ventilateur délivrant un gaz contenant de l'oxygène,
- un système de stockage et de distribution d'un mélange gazeux formé de NO/N₂ selon l'invention,
- un circuit patient relié fluidiquement au ventilateur, et
- un dispositif de distribution de NO permettant de contrôler la quantité de NO/N₂ provenant du système de stockage et de distribution et libérée dans le circuit patient.

En outre, l'invention concerne aussi un procédé de distribution d'un mélange NO/N₂, dans lequel on utilise un système de stockage et de distribution d'un mélange gazeux formé de NO/N₂ selon l'invention ou une installation de distribution d'un gaz contenant du NO à un patient selon l'invention.

La présente invention va maintenant être mieux comprise grâce à la description donnée ci-après en référence aux Figures annexées parmi lesquelles :
- la Figure 1 est un mode de réalisation d'une installation de distribution de NO alimentée par un système de conditionnement de gaz selon l'invention, et
- la Figure 2 est un schéma de principe de fonctionnement d'un robinet à détendeur intégré (RDI) d'un système de stockage et de distribution de gaz conforme à la présente invention.

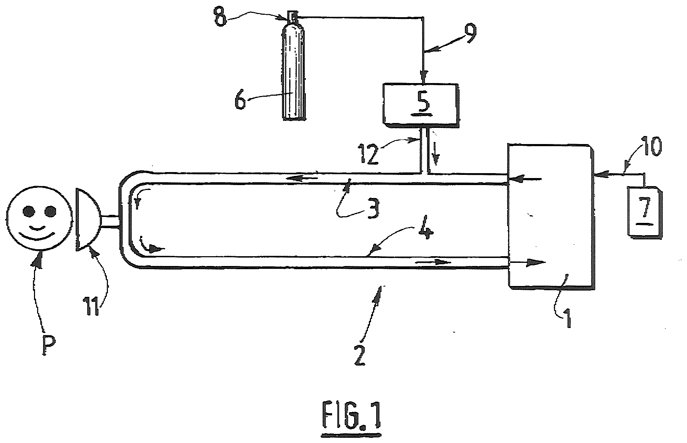

Le système de stockage et de distribution selon l'invention est utilisable pour alimenter une installation de distribution de NO à des patients souffrants de vasoconstrictions pulmonaires, par exemple via l'installation de distribution de NO dont un mode de réalisation est schématisé en Figure 1.

Cette installation comprend un ventilateur 1 avec un circuit respiratoire ou circuit patient 2 à deux branches, c'est-à-dire avec une branche inspiratoire 3 et une branche expiratoire 4. La branche inspiratoire 3 est conçue pour acheminer du gaz respiratoire du ventilateur 1 jusqu'au patient P, alors que la branche expiratoire 4 est conçue pour acheminer le gaz expiré par le patient P jusqu'au ventilateur 1.

Au niveau du patient P, l'administration du gaz se fait au moyen d'une interface patient 11, par exemple un masque respiratoire, une canule trachéale ou des lunettes nasales.

Le ventilateur 1 est alimenté, via une ligne de liaison 10, avec un gaz contenant de l'oxygène, par exemple de l'air (teneur en O₂ de 21% en volume) ou de l'oxygène issu d'une source d'oxygène 7, telle une bouteille d'oxygène ou une canalisation véhiculant de l'oxygène provenant d'une unité de production d'oxygène, telle une unité à pression modulée (PSA), ou d'une unité de stockage d'oxygène, telle un réservoir tampon ou de stockage.

Le gaz riche en oxygène est délivré par le ventilateur 1 dans la branche inspiratoire 3 du circuit patient 2.

Par ailleurs, un dispositif 5 de distribution de NO est relié fluidiquement à ladite branche inspiratoire 3 du circuit patient 2 pour y délivrer, via une ligne d'alimentation 12, un mélange NO/N₂, par exemple 200, 400, 800 ou 1500 ppmv de NO et le reste étant de l'azote.

Le dispositif 5 de distribution de NO est lui-même alimenté en mélange NO/N₂, via une ligne d'amenée de gaz 9, par un récipient 6 de NO/N₂ faisant partie d'un système de stockage et de distribution selon l'invention.

Le récipient 6 de NO/N₂ est une bouteille de gaz, par exemple en fibres composites, en aluminium ou en un alliage d'aluminium de contenant de 0.5, 2, 5, 10, 11 ou 20 litres (équivalent en eau), équipée d'un robinet à détendeur intégré 8, de préférence protégé par un capotage de protection contre les chocs.

Le robinet à détendeur intégré 8, appelé « RDI », permet de contrôler la sortie du gaz du récipient 6 et sa pression de sortie, notamment au moyen d'un clapet de détente 27 et d'un siège de clapet 28.

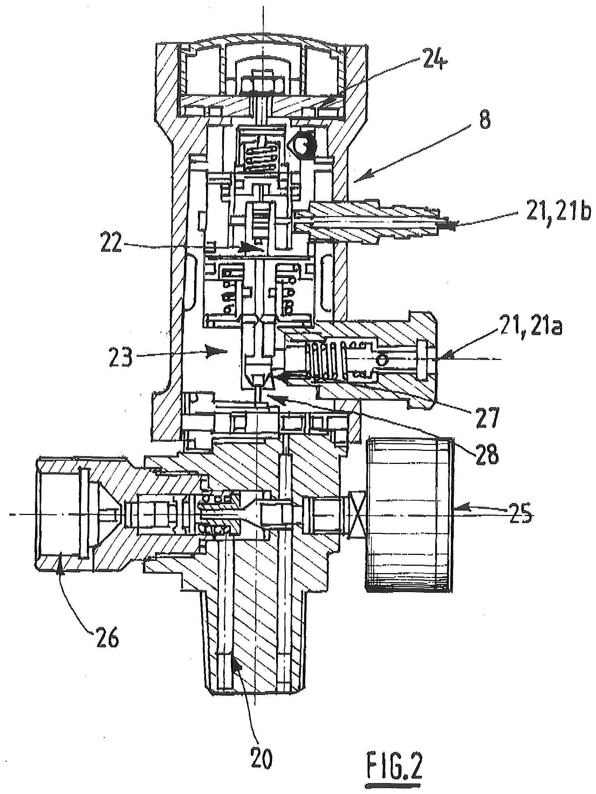

Ce RDI 8 se compose d'un corps parcouru par un ou plusieurs passages 22 de gaz reliant une entrée 20 de gaz située au niveau de l'extrémité de fixation du RDI sur le col de la bouteille à un ou plusieurs raccords 21 de sortie par lesquels le gaz ressort du RDI, après détente, comme détaillé ci-après en référence à la Figure 2.

La Figure 2 représente un mode de réalisation d'un robinet à détendeur intégré ou « RDI » en acier inoxydable conçu pour être monté sur un récipient de conditionnement de mélange gazeux de NO et d'azote, en particulier par vissage sur le col d'une bouteille de gaz.

Ce RDI 8 comprend un corps 23 de robinet (vu en coupe) comprenant un passage interne 22 de gaz reliant, d'une part, une entrée 20 de gaz par laquelle le mélange NO/azote est extrait du corps de la bouteille 6 de gaz, sur laquelle est monté ce RDI 8, et, d'autre part, plusieurs sorties 21 de gaz, c'est-à-dire des raccords de sortie de gaz, par laquelle le mélange NO/azote sort du RDI 8, avant d'être convoyé jusqu'au patient P, comme expliqué ci-dessus en référence à la Figure 1.

Selon l'invention, le RDI comprend deux raccords de sortie de gaz 21, 21a, 21b comprenant :
- un premier raccord 21a de sortie de gaz dit 'en pression', qui délivre du gaz à une basse pression typiquement comprise entre 1 et 10 bars, sur lequel viennent se connecter des appareils de délivrance du gaz. Le premier raccord 21 a de sortie de gaz est alimenté par du gaz provenant de la source de gaz à haute pression et ayant été détendu par les moyens de détente du RDI, en particulier le clapet et le siège de clapet.
- et un second raccord 21b de sortie à débit donné qui délivre des débits de gaz sélectionnés correspondant à des volumes prédéterminés de manière à fournir des débits de gaz différents, par exemple de 5 à 20 débits différents, typiquement de 10 à 15 débits différents, qui sont choisis par l'utilisateur en fonction du patient considéré, par exemple adulte ou enfant. Le second raccord de sortie permet donc une délivrance de NO gazeux à des débits de gaz différents qui sont sélectionnables au moyen de plusieurs orifices calibrés de diamètres croissants. Les orifices calibrés de diamètres différents font parmi d'un dispositif ou système de type débitlitre. De préférence, les orifices calibrés sont aménagés sur un disque rotatif agencé sur le passage du gaz en amont du second raccord 21b de sortie à débit donné. Le second raccord 21b de sortie permet donc de délivrer du gaz à différentes valeurs de débit comprises entre 50 ml/min et 5 l/min, typiquement entre 75 ml/min et 2 l/min, en particulier entre 100 ml/min et 1,5 l/min.

Un ou des organes de commande 24, tel un organe rotatif manipulable par un opérateur, permettent de contrôler la libération du gaz et de sélectionner le débit désiré. Il s'agit donc d'un sélecteur de débit gazeux, par exemple un bouton ou volant rotatif.

En particulier, un organe de commande 24 de débit agit sur le disque rotatif afin de permettre d'assurer une sélection de la valeur de débit désirée délivrée par le second raccord 21b de sortie

Afin de minimiser la corrosion des éléments du RDI 8 du fait de la nature corrosive du mélange gazeux NO/azote, le corps 23 du RDI 8 est en tout ou en partie, mais préférentiellement entièrement, réalisé en acier inoxydable.

En fait, on utilise de l'acier inoxydable en tant que matériau constitutif des passages et des autres éléments en contact avec le gaz à base de NO. Ainsi, le ou les passages de gaz sont en particulier percés à travers des parties en acier inoxydable du corps 23 du RDI 8.

De même, le clapet 27 de détente et/ou le siège 28 de clapet, et/ou d'autres éléments, sont aussi en acier inoxydable.

Le RDI 8 comporte en outre un manomètre 25 et un raccord de remplissage 26 permettant de remplir la bouteille 6 avec du gaz lorsque celle-ci est vide, c'est-à-dire que tout le gaz a été consommé. Le RDI 8 comprend ou peut comprendre par ailleurs d'autres composants classiques, tel que ressorts, joints d'étanchéité...

En outre, le dispositif 5 de distribution de NO permet notamment de contrôler la quantité de NO/N₂ libérée dans la branche inspiratoire 3, ainsi que le mode de libération de ce mélange, c'est-à-dire en continu ou de façon pulsée, par exemple uniquement pendant les phases inspiratoires du patient P. Il s'opère donc dans la branche inspiratoire 3, une dilution du mélange NO/N₂ avec le gaz riche en O₂ distribué par le ventilateur 1. La dilution est fonction de la teneur du mélange NO/N₂ initial mais aussi de la concentration de gaz à administrer au patient.

## Revendications

1. Système de stockage et de distribution d'un mélange gazeux formé de NO/N₂ comprenant :
- un récipient (6) de conditionnement de gaz contenant un mélange gazeux formé de NO et d'azote, et
- un robinet à détendeur intégré (8) comprenant un corps (D) de robinet en métal comprenant une entrée (20) de gaz, au moins une sortie (21 ; 21a, 21b) de gaz et au moins un passage interne gaz (22) traversant ledit corps (23) de robinet pour relier fluidiquement l'entrée (20) de gaz à ladite au moins une sortie de gaz (21; 21a, 21b), ledit robinet à détendeur intégré (8) étant fixé sur le récipient (6) de conditionnement,
**caractérisé en ce que** :
- au moins la partie du corps de robinet (23) traversée par ledit passage interne (22) de gaz est en acier inoxydable, et
- le robinet à détendeur intégré (8) comprend en outre :
. des moyens de réglage de la détente du gaz coopérant avec un clapet (27) de détente, ledit clapet (27) coopérant avec un siège (28) de clapet, le clapet (27) de détente et le siège (28) de clapet étant en acier inoxydable,
. un premier raccord (21a) de sortie dite 'en pression' qui délivre du gaz à basse pression comprise entre 1 et 10 bars, et
. un second raccord (21b) de sortie de débit associé à un dispositif débitlitre comprenant des orifices calibrés et un organe de sélection de débit pour permettre une délivrance du mélange gazeux NO/azote à plusieurs débits de gaz différents.

2. Système selon la revendication précédente, **caractérisé en ce que** le corps (23) du robinet est entièrement en acier inoxydable.

3. Système selon l'une des revendications précédentes, **caractérisé en ce que** le récipient (6) de conditionnement est de forme cylindrique et comprend un fond et un col avec un orifice de sortie au niveau duquel est fixé le récipient (6) de conditionnement, de préférence le robinet à détendeur intégré (8) est protégé par un capotage protection.

4. Système selon l'une des revendications précédentes, **caractérisé en ce que** le robinet (8) comprenant de 2 à 20 orifices calibrés ayant des diamètres différents les uns des autres.

5. Système selon l'une des revendications précédentes, **caractérisé en ce que** le robinet à détendeur intégré (8) comprend en outre des moyens de contrôle de la libération du gaz.

6. Système selon l'une des revendications précédentes, **caractérisé en ce que** les moyens de réglage de la détente du gaz et/ou les moyens de contrôle de la libération du gaz comprennent un ou des organes rotatifs (24) actionnables manuellement par un utilisateur.

7. Système selon l'une des revendications précédentes, **caractérisé en ce que** les orifices calibrés sont agencés en amont du second raccord de sortie (21b) de manière à ce que le gaz traverse l'un de ces orifices calibrés avant d'atteindre le second raccord de sortie (21b).

8. Système selon l'une des revendications précédentes, **caractérisé en ce que** les orifices calibrés ont des diamètres croissants correspondant aux différentes valeurs de débits désirés.

9. Système selon l'une des revendications précédentes, **caractérisé en ce que** le récipient (6) de conditionnement à un volume interne inférieur ou égal à 20 litres (équivalent en eau).

10. Système selon l'une des revendications précédentes, **caractérisé en ce qu'**il comporte un dispositif de suivi de l'autonomie de la bouteille en volume (litres) et/ou en temps (h ou min).

11. Système selon l'une des revendications précédentes, **caractérisé en ce que** les orifices calibrés ont des diamètres croissants correspondant à des valeurs de débits désirés compris entre 50 ml/min et 5 l/min, typiquement entre 75 ml/min et 2 l/min.

12. Système selon l'une des revendications précédentes, **caractérisé en ce que** les orifices calibrés sont aménagés sur un disque rotatif agencé sur le passage du gaz en amont du second raccord de sortie (21b).

13. Système selon l'une des revendications précédentes, **caractérisé en ce que** le raccord de sortie pour le gaz à basse pression comprend des moyens de détrompage.

14. Installation de distribution d'un gaz contenant du NO à un patient comprenant :
- un ventilateur (1) délivrant un gaz contenant de l'oxygène,
- un système de stockage et de distribution d'un mélange gazeux formé de NO/N₂ selon l'une des revendications précédentes,
- un circuit patient (2) relié fluidiquement au ventilateur (1), et
- un dispositif (5) de distribution de NO permettant de contrôler la quantité de NO/N₂ provenant du système de stockage et de distribution et libérée dans le circuit patient (2).

15. Procédé de distribution d'un mélange NO/N₂, dans lequel on utilise un système de stockage et de distribution d'un mélange gazeux formé de NO/N₂ selon l'une des revendications 1 à 13 ou une installation de distribution d'un gaz contenant du NO à un patient selon la revendication 14.

## Patentansprüche

1. System zum Speichern und zum Verteilen eines aus NO/N₂ gebildeten Gasgemisches, umfassend:
- einen Behälter (6) zur Gaskonditionierung, der ein Gasgemisch enthält, das aus NO und aus Stickstoff gebildet wird, und
- ein integriertes Druckminderventil (8), einen Ventilkorpus (D) aus Metall umfassend, der einen Gaseinlass (20), mindestens einen Gasauslass (21; 21a; 21b) und mindestens einen internen Gasdurchgang (22) umfasst, der den Ventilkorpus (23) durchquert, um den Gaseinlass (20) fluidmäßig mit dem mindesteins einen Gasauslass (21; 21a; 21b) zu verbinden, wobei das integrierte Druckminderventil (8) auf dem Behälter (6) zur Konditionierung befestigt ist,
**dadurch gekennzeichnet, dass**:
- mindestens der Abschnitt des Ventilkorpus (23), der durch den internen Gasdurchgang (22) durchquert wird, aus nichtrostendem Stahl ist, und
- das integrierte Druckminderventil (8) weiter umfasst:
. Mittel zum Einstellen der Druckminderung des Gases, die mit einer Druckminderklappe (27) zusammenwirken, wobei die Klappe (27) mit einem Klappensitz (28) zusammenwirkt, wobei die Druckminderklappe (27) und der Klappensitz (28) aus nichtrostendem Stahl sind,
. einen ersten ,druckbeaufschlagt' genannten Auslassanschluss (21a), der Gas mit niedrigem Druck zwischen 1 und 10 bar abgibt, und
. einen zweiten Durchflussmengen-Auslassanschluss (21b), der einer Durchflussmessvorrichtung zugeordnet ist, kalibrierte Öffnungen und ein Organ zur Durchflussmengenauswahl umfassend, um eine Abgabe des NO/Stickstoff Gasgemisches mit mehreren unterschiedlichen Gas-Durchflussmengen zu ermöglichen.

2. System nach dem vorstehenden Anspruch, **dadurch gekennzeichnet, dass** der Korpus (23) des Ventils vollkommen aus nichtrostendem Stahl ist.

3. System nach einem der vorstehenden Ansprüche, **dadurch gekennzeichnet, dass** der Behälter (6) zur Konditionierung in einer zylindrischen Form ist und einen Boden und einen Hals mit einer Auslassöffnung umfasst, in deren Bereich der Behälter (6) zur Konditionierung befestigt ist, wobei das integrierte Druckminderventil (8) vorzugsweise durch eine Schutzabdeckung geschützt ist.

4. System nach einem der vorstehenden Ansprüche, **dadurch gekennzeichnet, dass** das Ventil (8) 2 bis 20 kalibrierte Öffnungen umfasst, die voneinander unterschiedliche Durchmesser aufweisen.

5. System nach einem der vorstehenden Ansprüche, **dadurch gekennzeichnet, dass** das integrierte Druckminderventil (8) weiter Mittel zur Kontrolle der Freigabe des Gases umfasst.

6. System nach einem der vorstehenden Ansprüche, **dadurch gekennzeichnet, dass** die Mittel zum Einstellen der Druckminderung des Gases und/ oder die Mittel zur Kontrolle der Freigabe des Gases ein oder mehrere Drehorgane (24) umfassen, die von Hand durch einen Benutzer betätigt werden können.

7. System nach einem der vorstehenden Ansprüche, **dadurch gekennzeichnet, dass** die kalibrierten Öffnungen stromaufwärts des zweiten Auslassanschlusses (21b) angeordnet sind, damit das Gas eine dieser kalibrierten Öffnungen durchquert, bevor es den zweiten Auslassanschluss (21b) erreicht.

8. System nach einem der vorstehenden Ansprüche, **dadurch gekennzeichnet, dass** die kalibrierten Öffnungen entsprechend den gewünschten unterschiedlichen Durchflussmengenwerten zunehmende Durchmesser aufweisen.

9. System nach einem der vorstehenden Ansprüche, **dadurch gekennzeichnet, dass** der Behälter (6) zur Konditionierung ein Innenvolumen kleiner oder gleich 20 Litern (Wasseräquivalent) aufweist.

10. System nach einem der vorstehenden Ansprüche, **dadurch gekennzeichnet, dass** es eine Vorrichtung zur Überwachung der Autonomie der Flasche in Volumen (Liter) und/ oder in Zeit (Std. oder Min.) umfasst.

11. System nach einem der vorstehenden Ansprüche, **dadurch gekennzeichnet, dass** die kalibrierten Öffnungen entsprechend den gewünschten Durchflussmengenwerten zwischen 50 ml/min und 5 l/min, typischerweise zwischen 75 ml/min und 2 l/min, zunehmende Durchmesser aufweisen.

12. System nach einem der vorstehenden Ansprüche, **dadurch gekennzeichnet, dass** die kalibrierten Öffnungen auf einer Drehscheibe eingerichtet sind, die auf dem Durchgang des Gases stromaufwärts des zweiten Auslassanschlusses (21b) angeordnet ist.

13. System nach einem der vorstehenden Ansprüche, **dadurch gekennzeichnet, dass** der Auslassanschluss für das Gas mit niedrigem Druck Mittel zur Verwechslungssicherung umfasst.

14. Installation zum Verteilen eines NO enthaltenden Gases an einen Patienten, umfassend:
- einen Lüfter (1), der ein Sauerstoff enthaltendes Gas abgibt,
- ein System zum Speichern und zum Verteilen eines aus NO/N₂ gebildeten Gasgemisches nach einem der vorstehenden Ansprüche,
- einen Patientenkreislauf (2), der fluidmäßig mit dem Lüfter (1) verbunden ist, und
- eine Vorrichtung (5) zum Verteilen von NO, das es erlaubt, die Menge an NO/N₂ zu kontrollieren, die aus dem System zum Speichern und zum Verteilen stammt und in den Patientenkreislauf (2) freigegeben wird.

15. Verfahren zum Verteilen eines NO/N₂ Gemisches, wobei man ein System zum Speichern und zum Verteilen eines aus NO/N₂ gebildeten Gasgemisches nach einem der Ansprüche 1 bis 13, oder eine Installation zum Verteilen eines NO enthaltenden Gases an einen Patienten nach Anspruch 14 verwendet.

## Claims

1. System for storing and distributing a gaseous mixture formed of NO/N₂ comprising:
- a container (6) for processing gas containing a gaseous mixture formed of NO and nitrogen, and
- an integrated valve and regulator (8) comprising a metal valve body (D) comprising a gas inlet (20), at least one gas outlet (21; 21a, 21b) and at least one internal gas passage (22) crossing said valve body (23) to smoothly connect the gas inlet (20) to said at least one gas outlet (21; 21a, 21b), said integrated valve and regulator (8) being attached to the processing container (6),
**characterised in that**:
- at least the part of the valve body (23) crossed by said internal gas passage (22) is made of stainless steel, and
- the integrated valve and regulator (8) further comprises:
-- means for regulating the expansion of the gas cooperating with an expansion valve (27), said valve (27) cooperating with a valve seat (28), the expansion valve (27) and the valve seat (28) being made of stainless steel,
-- a first outlet connector (21a) known as 'pressurised' which delivers the low-pressure gas of between 1 and 10 bars, and
--a second outlet flow connector (21b) connected to a flowmeter device comprising calibrated orifices and a flow selection body to allow a delivery of the NO/nitrogen gaseous mixture to several different gas flows.

2. System according to the preceding claim, **characterised in that** the body (23) of the valve is entirely made of stainless steel.

3. System according to one of the preceding claims, **characterised in that** the processing container (6) is of a cylindrical shape, and comprises a bottom and a collar with an outlet orifice at the level of which the processing container (6) is attached, preferably the integrated valve and regulator (8) is protected by a protective enclosure.

4. System according to one of the preceding claims, **characterised in that** the valve (8) comprising from 2 to 20 calibrated orifices that have different diameters to each other.

5. System according to one of the preceding claims, **characterised in that** the integrated valve and regulator (8) further comprises gas control and release means.

6. System according to one of the preceding claims, **characterised in that** the means for regulating the expansion of the gas and/or the means for controlling the release of the gas comprise one or several rotating bodies (24), actionable manually by a user.

7. System according to one of the preceding claims, **characterised in that** the calibrated orifices are arranged above the second outlet connector (21b) so that the gas crosses one of these calibrated orifices before reaching the second outlet connector (21b).

8. System according to one of the preceding claims, **characterised in that** the calibrated orifices have increasing diameters corresponding to the different values of desired flows.

9. System according to one of the preceding claims, **characterised in that** the processing container (6) has an internal volume of less than or equal to 20 litres (water equivalent).

10. System according to one of the preceding claims, **characterised in that** it comprises a device for monitoring the autonomy of the bottle in volume (litres) and/or in time (hours or minutes).

11. System according to one of the preceding claims, **characterised in that** the calibrated orifices have increasing diameters corresponding to the different values of desired flows between 50ml/min and 5l/min, typically between 75ml/min and 2l/min.

12. System according to one of the preceding claims, **characterised in that** the calibrated orifices are arranged on a rotating disc arranged on the gas passage above the second outlet connector (21b).

13. System according to one of the preceding claims, **characterised in that** the outlet connector for the low-pressure gas comprises keyed connection means.

14. Installation for distributing a gas containing NO to a patient comprising:
- a ventilator (1) delivering a gas containing oxygen,
- a system for storing and distributing a gaseous mixture formed of NO/N₂ according to one of the preceding claims,
- a patient circuit (2) smoothly connected to the ventilator (1), and
- a device (5) for distributing NO allowing to control the quantity of NO/N₂ coming from the storage and distribution system and released in the patient circuit (2).

15. Method for distributing an NO/N₂ mixture, wherein a system for storing and distributing a gaseous mixture formed of NO/N₂ is used, according to one of the claims 1 to 13 or an installation for distributing a gas containing NO to a patient according to claim 14.
